# EUROPEAN PATENT APPLICATION

(11) **EP 1 454 911 A1**
(43) Date of publication of application: **08.09.2004**
(21) Application number: 03005174.2
(22) Date of filing: 07.03.2003
(51) Int. Cl.: C07F 9/09, A61K 6/08, C08F 30/02

(54) **A polymerizable phosphoric acid ester derivative and a dental composition employing it**

(71) Applicant: DENTSPLY DETREY GmbH, 78467 Konstanz (DE)
(72) Inventor: Klee, Joachim, E, 78315 Radolfzell (DE); Lehmann, Uwe, 78467 Konstanz (DE); Walz, Uwe, 78467 Konstanz (DE); Liu, Huaibing, Dover, DE 19901 (US)
(74) Representative: Hartz, Nikolai F., Dr.

(57) **Abstract**

The present invention provides a polymerizable phosphoric acid ester derivative having the following formula (A): 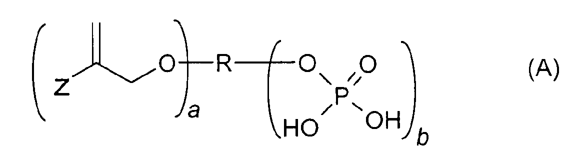 wherein
Z is COOR₁, COSR₁, CON(R₁)₂, CONR₁R₂, CONHR₁, or CN,
R₁ and R₂ independently are a hydrogen atom, a substituted or unsubstituted C₁ to C₁₈ alkyl group optionally substituted by a C₃ to C₈ cycloalkyl group, a substituted or unsubstituted C₃ to C₈ cycloalkyl group, a substituted or unsubstituted C₄ to C₁₈ aryl or heteroaryl group, a substituted or unsubstituted C₅ to C₁₈ alkylaryl or alkylheteroaryl group, or a substituted or unsubstituted C₇ to C₃₀ aralkyl group, whereby two R₁ residues may form together with the N-atom to which they are bonded a 5- to 7-membered heterocyclic ring which may contain beside said N-atom a further nitrogen atom or an oxygen atom, and whereby the substituted groups may be substituted by 1 to 5 C₁ to C₅ alkyl group(s);
*a* is an integer of from 1 to 10, preferably 1 to 5;
*b* is an integer of from 1 to 10, preferably 1 to 5; and
R represents an organic residue containing *a* + *b* carbon atoms selected from primary and secondary aliphatic carbon atoms, secondary alicyclic carbon atoms, and aromatic carbon atoms, each of said carbon atoms linking a phosphate or 2-(oxa-ethyl)acryl derivative group.

## Description

The present invention relates to a polymerizable phosphoric acid ester derivative, its use in a dental composition, a process for its production, and a dental composition employing it. More specifically the polymerizable phosphoric acid ester derivative, due to its stability against hydrolysis in acidic medium, allows the provision of a one-part self-etching, self-priming dental adhesive composition.

### Technical background

Presently, self-etching, self-priming dental adhesives are composed of two-part systems due to low hydrolysis stability of the polymerizable acidic monomers. The low hydrolysis stability arises from the hydrolysis of acidic and adhesive monomers in water or water/solvent mixtures. Therefore, the known acidic and adhesive monomers must be stored water-free and mixed with the aqueous part just before application.

Frequently, sulfuric acid ester and phosphoric acid ester groups are employed in acidic polymerizable adhesive monomers. However, these acidic groups hydrolyze the acrylic and methacrylic ester moieties as well as the phosphoric acid ester groups within the monomers (Moszner et al. Macromol. Chem. Phys. **2000**, 1062, (1999), DE 199 18 974, EP 1 169 996). In order to overcome these disadvantages, polymerizable phosphonic ester monomers were proposed by Moszner et al. Macromol. Chem. Phys. **2000**, 1062, (1999), DE 199 18 974, and EP 1 169 996. Moreover, US 4,539,382 discloses mono(meth)acrylamides with one phosphonic acid group. However, these monomers still comprise hydrolysable (meth)acrylic ester moieties. Therefore, monomers with phosphonic acid ester groups based on 2-(oxa alkyl) acrylate were suggested in DE 197 46 708. However, also these phosphonic acid derivatives tend to hydrolyse in acidic solution. Therefore, it has not been possible to provide an one-part self-etching, self-priming dental adhesive composition. An one-part composition means that the composition is contained in only one container which may be stored. It allows application of the composition without any mixing and without any special equipment before the application. Self-etching means that the dental adhesive composition may be applied to a tooth without any preliminarily etching of enamel in a separate method step. In order to comprise such a self-etching feature, the composition must be acidic. Self-priming means that the dental adhesive composition may be applied to a tooth without any preliminarily application of a primer.

In practice the monomers of the prior art could be employed only in two-part dental systems which consist of a priming part and a bonding part. These two-part dental adhesive systems are either applied sequentially or in one step after mixing the two parts. Both procedures have inherent disadvantages due to clinical complications which might occur between sequential steps (saliva or blood contamination) or due to dosing problems when mixing is required prior to the application of the self-etching adhesive.

In order to overcome these clinical problems it is desired to provide a self-priming and self-etching adhesive as a one-part system eliminating the need of sequential application or premixing.

Further disadvantages of the monomers of the prior art containing phosphonic acid derivatives are as follows: The phosphonic acids are less acidic than phosphoric acid. Therefore, additional acids are required for obtaining the self-etching feature of a dental composition. However, the additional acid increases generally degradation of the monomer by hydrolysis. Moreover, the intermediates for producing the phosphonic acid derivatives are toxic. Therefore, the process for the preparation is dangerous and more complicated. Further, the phosphonic ester derivatives are more expensive than phosphoric acid derivatives.

### Description of the invention

The above described needs and the disadvantages of the polymerizable phosphonic acid derivatives are solved by the polymerizable phosphoric acid ester derivative having the following formula (A): 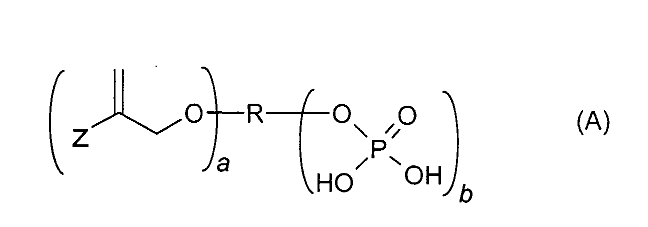 wherein
Z is COOR₁, COSR₁, CON(R₁)₂, CONR₁R₂, CONHR₁, or CN,
R₁ and R₂ independently are a hydrogen atom, a substituted or unsubstituted C₁ to C₁₈ alkyl group optionally substituted by a C₃ to C₈ cycloalkyl group, a substituted or unsubstituted C₃ to C₈ cycloalkyl group, a substituted or unsubstituted C₄ to C₁₈ aryl or heteroaryl group, a substituted or unsubstituted C₅ to C₁₈ alkylaryl or alkylheteroaryl group, or a substituted or unsubstituted C₇ to C₃₀ aralkyl group, whereby two R₁ residues may form together with the N-atom to which they are bonded a 5- to 7-membered heterocyclic ring which may contain beside said N-atom a further nitrogen atom or an oxygen atom, and whereby the substituted groups may be substituted by 1 to 5 C₁ to C₅ alkyl group(s);
*a* is an integer of from 1 to 10, preferably 1 to 5;
*b* is an integer of from 1 to 10, preferably 1 to 5; and
R represents an organic residue containing *a* + *b* carbon atoms selected from primary and secondary aliphatic carbon atoms, secondary alicyclic carbon atoms, and aromatic carbon atoms, each of said carbon atoms linking a phosphate or 2-(oxa-ethyl)acryl derivative group.

The organic residue R in the polymerizable phosphoric acid ester derivative of the present invention may contain further carbon, hydrogen, and hetero atoms, preferably oxygen and sulfur atoms, whereby oxygen atoms are particularly preferred. The number of the further atoms may vary and is not limited. According to a preferred embodiment of the present invention R may contain from 2 to 45, preferably up to 30, more preferably up to 18 and most preferably up to 10 carbon atoms. Also the number of further heteroatoms is not limited. According to a preferred embodiment of the invention R may contain from 1 to 10 heteroatom(s), preferably oxygen atom(s). Further, the amount of hydrogen atoms may vary. It depends on the amount of carbon and heteroatoms.

In this specification an aryl group may for example be a phenyl group, naphthyl group, anthryl group, etc., whereby a phenyl group representing a C₆ aryl group is a preferred aryl group. A heteroaryl group may for example be a furyl or thienyl group representing a C₄ heteroaryl group, whereby a thienyl group is particularly preferred.

The polymerizable phosphoric acid ester derivative of the present invention has surprisingly high hydrolysis stability, although a phosphate group is present. Surprisingly, it is hydrolysis stable both at the 2-(oxa-ethyl)-group and at the phosphoric acid ester group. It is hydrolysis stable under acidic conditions. Particularly, it is hydrolysis stable at a pH of at most 4, preferably at a pH of at most 2, most preferably at a pH of 1.0. Therefore, the polymerizable phosphoric acid ester derivative of the present invention allows the preparation of an advantageous one-pack self-etching and self-priming dental adhesive composition.

A one-pack composition means that the composition of the present invention is contained in only one container which may be stored and allows application of the composition without any mixing and without any special equipment before the application.

Self-etching means that the dental adhesive composition of the present invention may be applied to a tooth without any preliminarily etching of enamel in a separate method step. Particularly, the polymerizable phosphoric acid ester derivative of the present invention allows the preparation of a dental composition which is hydrolysis stable for at least one week at a storage temperature of 50 °C, whereby after such storage the bond strength of an adhesive prepared from such a dental composition to enamel and/or dentin is at least 10 MPa, preferably 15 MPa. Due to the high hydrolysis stability of the compound of the present invention a one-part self-etching and self-priming system which has excellent shelf-life may be prepared.

Further advantages of the polymerizable phosphoric acid ester derivative of the present invention are as follows: the phosphoric acid derivatives are stronger acidic than phosphonic acid derivatives. Therefore, it is not necessary to incorporate additional acids for obtaining the self-etching feature of a dental composition. However, a dental composition according to the present invention may include also further acids. They allow an easy adjustment of the pH. Surprisingly, neither the stronger phosphoric acid derivative of the invention nor additional acid(s) decrease the hydrolysis stability. Moreover, the intermediates for producing the phosphoric acid derivatives are not toxic. Therefore, the process for the preparation is safe, and the process for preparing the polymerizable phosphoric acid ester derivative of the present invention can be conducted more easily. Further, the phosphoric ester derivatives are generally less expensive than phosphonic acid derivatives.

In a preferred embodiment of the invention the organic residue R is an (*a*+*b*)-valent saturated aliphatic C₂ to C₁₈ group having at least 2 of said primary aliphatic carbon atoms, and optionally 1 or more of said secondary aliphatic carbon atom(s), whereby said (*a*+*b*)-valent group may be substituted by C₁ to C₅ alkyl group(s); or a C₂ to C₄₅ mono-, di-, or polyether which has from 1 to 14 oxygen atoms and is substituted by at least 2 C₁ to C₁₀ aliphatic group(s) having said primary and/or secondary aliphatic carbon atoms; whereby said ether may optionally be substituted by C₁ to C₅ alkyl group(s). The amount of these C₁ to C₅ alkyl group(s) may vary. Preferably, the ether may be substituted by 1 to 15, more preferably 1 to 5 C₁ to C₅ alkyl group(s).

According to a further preferred embodiment of the present invention, the organic residue R represents
a saturated C₃ to C₈ cyclic, C₇ to C₁₅ bi- or polycyclic hydrocarbon group having from 0 to 4, preferably, 0 to 3, more preferably 0 or 1, of said secondary alicyclic carbon atoms; and/or
a C₄ to C₁₈ aryl or heteroaryl group having from 0 to 5, preferably 0 to 3, more preferably 0 or 1, of said aromatic carbon atoms;
whereby said saturated hydrocarbon or aryl or heteroaryl group is substituted by
from 0 to 5 C₁ to C₅ alkyl group(s);
from 0 to 4, preferably 1 to 3, more preferably 1 or 2, saturated C₁ to C₁₀ aliphatic group(s) having said primary and/or secondary aliphatic carbon atoms, and/or
from 0 to 2 divalent residues according to one of the following formulas:
-[O-CH₂CH₂-]_{f}- wherein f is an integer of from 1 to 10, preferably 1 to 5;
-[-O-CH₂CH₂ CH₂-]_{g}- wherein g is an integer of from 1 to 10, preferably 1 to 5;
-[O-R₁₂]ₕ- wherein R₁₂ is -CH(CH₃)-CH₂- or -CH₂-CH(CH₃)- and h is an integer of from 1 to 10, preferably 1 to 5;
-[-O-R₁₄]ᵢ-[O-R₁₅]ⱼ- or -[O-R₁₅]ₖ [O-R₁₄]ₗ- wherein R₁₄ is -CH₂CH₂-, R₁₅ is -CH(CH₃)-CH₂- or -CH₂-CH(CH₃)-, i, j, k, and l are integers whereby
2i + 3j ≤ 15 and 2k + 3l ≤ 15,
-[O-CH₂CH₂CH₂CH₂-]ᵣ- wherein r is an integer of 1 or 2;
wherein said divalent residues have one of said primary aliphatic carbon atoms; and
whereby 2 groups selected from said saturated hydrocarbon, aryl, and heteroaryl groups may optionally be linked by a single bond, an alkylene group, or -O-. Said alkylene group may be a C₁ to C₈ alkylene group. Preferably it is a C₁ to C₃ alkylene group, whereby an iso-propylene group is particularly preferred.

In a further embodiment of the present invention the organic residue R is
an (*a*+*b*)-valent saturated C₃ to C₈ cyclic or C₇ to C₁₅ bi- or tricyclic hydrocarbon group having at least 2 of said secondary alicyclic carbon atoms;
an (*a*+*b*)-valent saturated C₅ to C₁₈ aryl or heteroaryl group having from 2 to 6 of said aromatic carbon atoms;
an (*a*+*b*)-valent C₆ to C₁₈ alkylaryl or alkyl heteroaryl group having at least one of said aromatic carbon atoms, at least one of said secondary aliphatic carbon atoms, and optionally one of said primary aliphatic carbon atoms at the terminal end of the alkyl moiety of said alkylaryl or alkylheteroaryl group; or
an (*a*+*b*)-valent C₈ to C₃₀ aralkyl group having at least one of said primary aliphatic carbon atoms and at least one of said secondary aliphatic carbon atoms.

Particularly preferred is the polymerizable phosphoric acid ester derivative which has one of the following formulas: 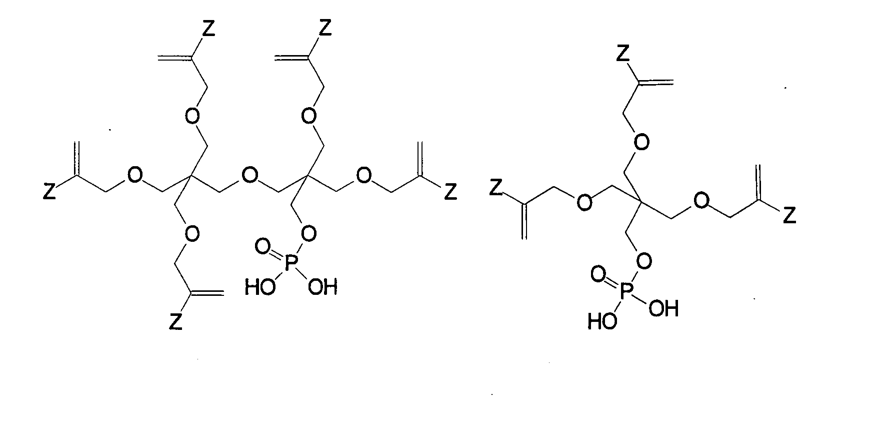 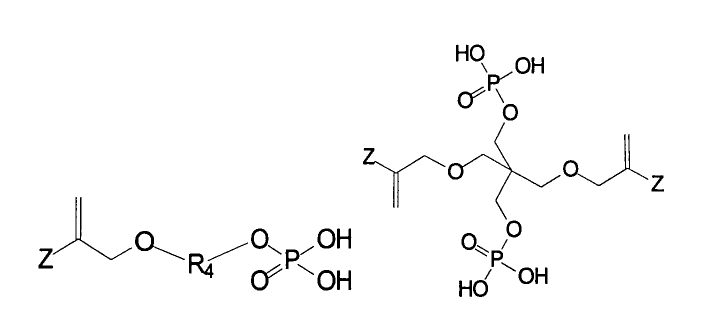 wherein
Z is as defined above,
R₄ denotes a divalent C₂ to C₁₈ alkylene group, a divalent C₃ to C₈ cycloalkylene group, a divalent C₄ to C₁₈ aryl or heteroaryl group, a divalent C₅ to C₁₈ alkylaryl or alkylheteroaryl group, a divalent C₇ to C₃₀ aralkyl group, whereby said groups may be substituted by 1 to 5 C₁ to C₅ alkyl group(s).

The polymerizable phosphoric acid ester derivatives in the above formulas have the advantage that a large number of polymerizable 2-(oxa-ethyl)acryl derivative groups and/or a large number of acidic phosphate groups are linked to one molecule. This allows to tailor the self-priming and self-etching features of a dental composition comprising such compounds. A large amount of polymerizable 2-(oxa-ethyl)acryl derivative groups per molecule enhances the bond strength of a dental adhesive composition comprising the polymerizable phosphoric acid ester derivative of the present invention. A large amount of phosphate groups per molecule enhances the self-etching feature of a dental composition comprising the polymerizable phosphoric acid ester derivative of the present invention.

In a further embodiment of the present invention, the polymerizable phosphoric acid ester derivative has the above formula wherein R₄ is a divalent residue according to one of the following formulas:
-[CH₂CH₂-O-]ₘ-CH₂CH₂- wherein m is an integer of from 1 to 14,
-[CH₂CH₂ CH₂-O-]ₚ-CH₂CH₂ CH₂- wherein p is an integer of from 1 to 14,
-[R₁₂-O]_{q}R₁₃- wherein R₁₂ and R₁₃ may be -CH(CH₃)-CH₂- or -CH₂-CH(CH₃)- and q is from 1 to 14,
-[R₁₄-O]ᵣ-[R₁₅-O]ₛ-R₁₄- or -[R₁₄-O]ₜ-[R₁₅-O]ᵤ-R₁₅- wherein R₁₄ is
-CH₂CH₂-, R₁₅ is -CH(CH₃)-CH₂- or -CH₂-CH(CH₃)-, r, s, t, and u are integers whereby 2r + 3s ≤ 43 and 2t + 3u ≤ 42,
-[CH₂CH₂CH₂CH₂-O-]ᵣ-CH₂CH₂CH₂CH₂- wherein r is 1 or 2, 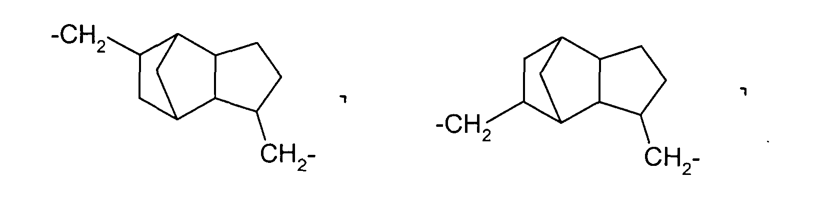 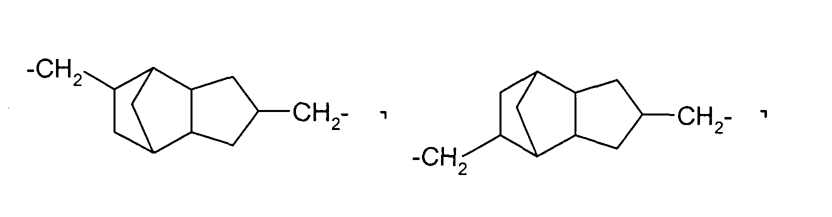 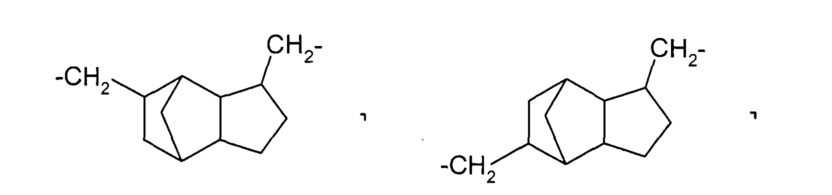 or 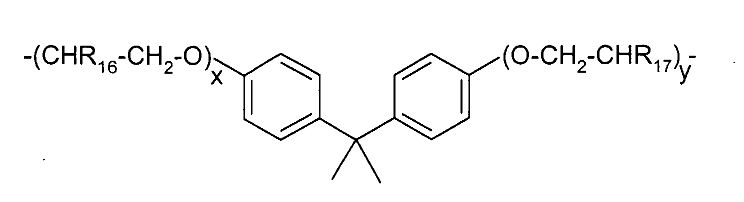 wherein R₁₆ and R₁₇ are H or -CH₃ and x and y may independently be integers of from 0 to 10, preferably 0 to 5.

Particularly preferred is the polymerizable phosphoric acid ester derivative as defined above, wherein said (*a*+*b*) carbon atoms are primary aliphatic carbon atoms.

The polymerizable phosphoric acid ester derivative of the present invention is hydrolysis stable under acidic conditions, preferably at a pH of at most 4, more preferably at a pH of at most 2, and most preferably at a pH of 1.0.

The polymerizable phosphoric acid ester derivative of the present invention may be prepared by a process which comprises the following steps:
(i) reacting a di- or polyol of the formula (HO)ₐ-R-(OH)_{b} (B) with a compound of the formula (C): 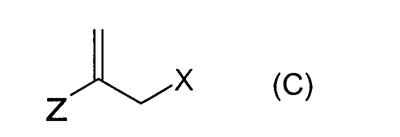 wherein Z, R, *a*, and *b* are as defined above, particularly wherein R is R₄ as defined above, and
   X is a leaving group for producing a compound (D) having *b* hydroxyl group(s) per molecule, and
(ii) reacting compound (D) with a phosphoric acid derivative (E) reactive with a hydroxyl group.

Preferably, the leaving group X is a halogen atom. Particularly preferred is that X is a chlorine or bromine atom, whereby a chlorine atom is most preferred.

The equivalent ratio between compound (C) and the di- or polyol (B) in the above process may be about *a* : 1. Further, the equivalent ratio between compounds (E) and (D) may be about *b* : 1. Preferably, the phosphoric acid derivative (E) is phosphorus trichloride oxide. Moreover, the above described method may comprise hydrolyzing the reaction product of compounds (D) and (E).

In case that a polyol is used as compound (B), it may be advantageous that *b* hydroxyl groups of the polyol (B) are protected, then reacted with compound (C) followed by deprotecting before conducting step (ii). Any known protection agent for protecting hydroxyl groups may be used. Particularly preferred is 3,4-dihydro-2*H*-pyrane, since it is suitable for primary, secondary and aromatic hydroxyl groups. Reacting 3,4-dihydro-2*H*-pyrane with an alcohol under acidic conditions results is a tetrahydropyranylether which is stable under basic conditions and may be cleaved or deprotected easily with mild acids.

Due to the excellent properties of the polymerizable phosphoric acid ester derivative it may be used in a dental composition. Such dental composition includes an adhesive, a primer, a cement, a composite, etc. Particular preferred is a one-part self-etching, self-priming dental adhesive composition.

Further, the present invention provides a dental composition comprising the polymerizable phosphoric acid ester derivative as described above.

In a preferred embodiment of the present invention, the dental composition is acidic. It may have a pH of at most 4, preferably a pH of at most 2, more preferably a pH of about 1.0.

Moreover the dental composition of the present invention may further comprise a curing system. Such a curing system may comprise a polymerization initiator, an inhibitor and a stabilizer. The polymerization initiator may be a thermal initiator, a redox-initiator or a photo initiator. Preferably, camphor quinone is used. A stabilizer may be applied in order to stabilize the dental composition. Such a stabilizer may for example be a radical absorbing monomer, such as hydroquinone monomethylether, 2,6-di-tert-butyl-p-cresol, tetramethyl piperidine N-oxyl radical, galvanoxyl radical.

In a preferred embodiment of the invention the dental composition may comprise a filler. This filler may be an inorganic filler and/or an organic filler. Preferably, the filler is a nanofiller.

Further, the dental composition of the present invention may comprise an organic water soluble solvent and/or water. The organic water soluble solvent may be selected from alcohols, such as ethanol, propanol, butanol; and/or ketones such as acetone and methyl ethyl ketone. Particularly preferred is acetone, ethanol and/or tert-butanol.

According to a further embodiment of the present invention, the dental composition may comprise an organic and/or inorganic acid. Preferably, the organic acid is a mono- or polycarboxylic acid, such as methacrylic acid, acrylic acid, fumaric acid, maleic acid, citric acid, itaconic acid, and/or formic acid; and the inorganic acid is preferably phosphoric acid, sulfuric acid and/or hydrofluoric acid.

The dental composition of the present invention may also comprise a polymerizable N-substituted alkylacrylic or acrylic acid amide monomer. Preferably, the polymerizable N-substituted alkylacrylic or acrylic acid amide monomer has one of the following formulas: 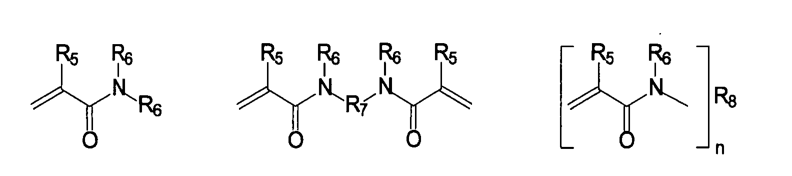 wherein
R₅ and R₆ independently represent an hydrogen atom, a substituted or unsubstituted C₁ to C₁₈ alkyl group, a substituted or unsubstituted C₃ to C₁₈ cycloalkyl group, a substituted or unsubstituted C₄ to C₁₈ aryl or heteroaryl group, a substituted or unsubstituted C₅ to C₁₈ alkylaryl or alkylheteroaryl group, a substituted or unsubstituted C₇ to C₃₀ aralkyl group, whereby two R₆ residues may form together with the N-atom to which they are bonded a 5- to 7-membered heterocyclic ring which may contain beside said N-atom a further nitrogen atom or an oxygen atom, and whereby the substituted groups may be substituted by 1 to 5 C₁ to C₅ alkyl group(s);
R₇ denotes a divalent substituted or unsubstituted organic residue having from 1 to 45 carbon atoms, whereby said organic residue may contain from 1 to 14 oxygen and/or nitrogen atoms and is selected from
a C₁ to C₁₈ alkylene group wherein from 1 to 6 -CH₂-groups may be replaced by a -N-(C=O)-CR₉=CH₂ group wherein R₉ is a hydrogen atom or a C₁ to C₁₈ alkyl group,
a divalent substituted or unsubstituted C₃ to C₁₈ cycloalkyl or cycloalkylene group,
a divalent substituted or unsubstituted C₄ to C₁₈ aryl or heteroaryl group,
a divalent substituted or unsubstituted C₅ to C₁₈ alkylaryl or alkylheteroaryl group,
a divalent substituted or unsubstituted C₇ to C₃₀ aralkyl group, and
a divalent substituted or unsubstituted C₂ to C₄₅ mono-, di- or polyether group having from 1 to 14 oxygen atoms,
R₈ denotes a saturated di- or multivalent substituted or unsubstituted C₂ to C₁₈ hydrocarbon group, a saturated di- or multivalent substituted or unsubstituted cyclic C₃ to C₁₈ hydrocarbon group, a di- or multivalent substituted or unsubstituted C₄ to C₁₈ aryl or heteroaryl group, a di- or multivalent substituted or unsubstituted C₅ to C₁₈ alkylaryl or alkylheteroaryl group, a di- or multivalent substituted or unsubstituted C₇ to C₃₀ aralkyl group, or a di- or multivalent substituted or unsubstituted C₂ to C₄₅ mono-, di-, or polyether residue having from 1 to 14 oxygen atoms,
n is an integer, preferably from 2 to 10, more preferably from 3 to 4.

More preferably, the dental composition of the present invention contains a mono-, bis- or poly(meth) acrylamide monomer characterized by one of the following formulas:

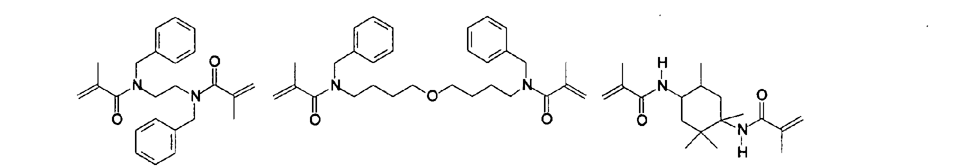

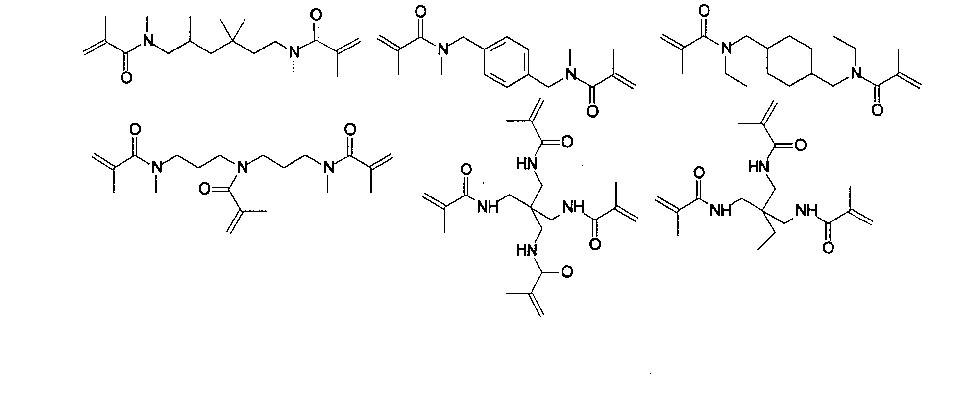

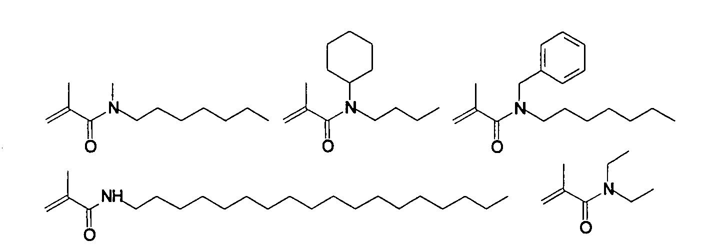

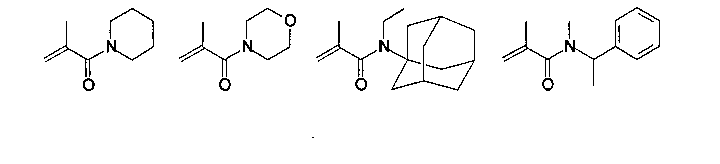

According to a particular preferred embodiment of the present invention, the dental composition is a hydrolysis stable one-part self-etching, self-priming dental adhesive composition. Such a composition is advantageously hydrolysis stable, e.g. for at least one week at a storage temperature of 50 °C, whereby after such storage the bond strength of an adhesive prepared from such a dental composition to enamel and/or dentin is at least 10 MPa, preferably 15 MPa.

The dental composition may contain from 5 to 90 wt-% of the polymerizable phosphoric acid ester derivative of the present invention.

The present invention will now be explained in further detail by the following examples.

### Example 1

### 2,2,2-Tris[2,6-dioxa-4-methylene-5-oxo-octyl]ethanol (1)

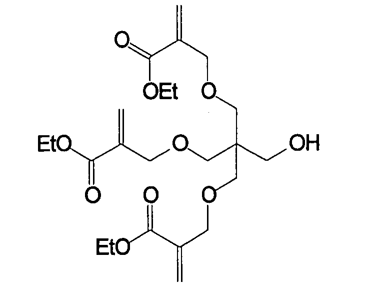

To a stirred solution of 2 g (14.68 mmol) pentaerythrit and 4.46 g (44.07 mmol) triethylamine in 100 ml tetrahydrofurane 6.56 g (44.14 mmol) ethyl chloromethylacrylate were added at room temperature. The reaction mixture was refluxed for 4 days. Filtration of the reaction mixture, evaporation of the solvent and drying under high vacuum yielded 6.76 g (yield: 97 %) of a clear, yellowish oil.

### 2,2,2-Tris[2,6-dioxa-4-methylene-5-oxo-octyl]ethanol phosphoric acid (2)

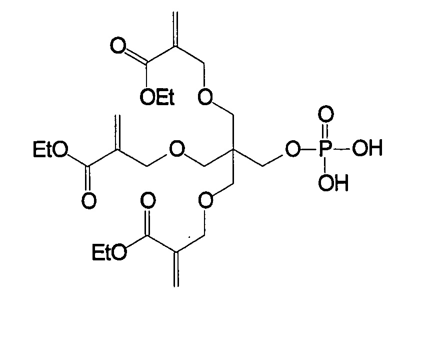

To a stirred solution of 1.112 g (7.30 mmol) phosphorusoxytrichloride in 10 ml tetrahydrofurane a solution of 3 g (6.34 mmol) 2,2,2-Tris[2,6-dioxa-4-methylene-5-oxo-octyl]ethanol **1** and 0.772 g (7.62 mmol) triethylamine in 30 ml tetrahydrofurane was added dropwise, while the temperature was kept at 0 °C. After the addition was finished the reaction mixture was stirred for 0.5 h at 0 °C and for 4 h at room temperature before the reaction was finished by filtration of the suspension and evaporation of the solvent. The oily residue was solved in 100 ml diethylether and added dropwise to 50 ml water, while the temperature was kept at 0 °C. The layers were separated and the organic solution was washed successively with 50 ml of an aqueous sodium hydroxide (25 wt%) solution and 300 ml of an aqueous hydrogen chloride (18 wt%) solution. The acidic extract was washed again with 100 ml diethylether and the unified organic fractions were dried over magnesium sulfate. Filtration and evaporation yielded 1.88 g (yield: 54 %) of a clear, yellowish oil.

### Example 2

### Ethyl 2-[12-hydroxy-2-oxadodecyl]acrylate (3)

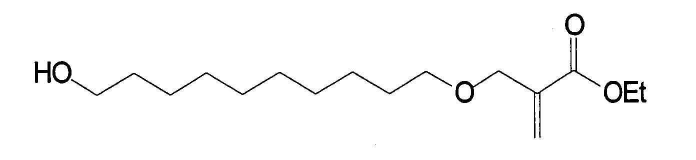

To a stirred solution of 10 g (57.4 mmol) 1,10-decandiol and 5.86 g (57.90 mmol) triethylamine in 100 ml tetrahydrofurane 8.50 g (57.20 mmol) ethyl α-chloromethylacrylate were added at room temperature. The reaction mixture was refluxed for 40 h. The reaction mixture was cooled down to room temperature and the developing precipitate was filtered off. Evaporation of the filtrate afforded a mixture of a clear, yellow oil with a white solid. This mixture was taken up in a small amount of dichloromethane and filtered again. The filtrate was solved in 150 ml dichloromethane and washed twice with 50 ml water. The organic layer was dried over magnesium sulfate. Then the mixture was filtered, and the solvent was evaporated. The crude product was purified by column chromatography on silica gel with dichloromethane and ethyl acetate as elution agents. This afforded 6,37 g (yield: 38,9 %) of a clear, yellowish oil.

IR(film, cm⁻¹) 3425 (OH), 2926/2855 (CH₃/CH₂), 1714 (CO), 1638 (C=C), 1459/1375/1303 (CH₃/CH₂), 1270/1172/1102/1031/949.
¹H-NMR (250 MHz, CDCl₃, ppm) 1.08-1.24 (m, 15H, CH₂,CH₃), 1.24-1.49 (m, 4H, CH₂), 3.17 (broad s, 1H, OH), 3.27 (t, 2H, OCH₂) 3.37 (t, 2H, OCH₂), 3.96 (s, 2H, CH₂(1)), 4.01 (q, 2H, OC*H*₂CH₃) 5.65 (s, 1H, C*H*=C), 6.07 (s, 1H, C*H*=C).
¹³C-NMR (63 MHz, CDCl₃, ppm) 13.64 (CH₃), 25.36, 25.68, 28.98, 29.07, 29.11, 19.17 and 32.24 (CH₂ (4-11)), 60.10 and 61.90 (CH₂ (12) and CH₂CH₃), 68.31 and 70.52 (CH₂ (1) and CH₂ (3)), 124.69 (*C*=C-CO), 137.12 (C=*C*-CO), 165.37 (C=C-*C*O).

### Ethyl 2-[12-dihydrogen phosphoryl-12,2-dioxatridecyl]acrylate (4)

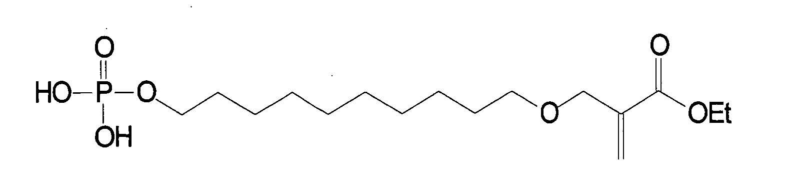

To a stirred solution of 3.21 g (20.94 mmol) phosphorusoxytrichloride in 50 ml diethylether a solution of 5 g (17.45 mmol) **3** and 2.22 g (20.94 mmol) triethylamine in 50 ml diethylether was added dropwise, while the temperature was kept at 0 °C. After the addition was finished the reaction mixture was stirred for 0.5 h at 0 °C and for 16 h at room temperature before the reaction was finished by filtration of the suspension and evaporation of the solvent. The oily residue was solved in 100 ml diethylether and added dropwise to 100 ml water, while the temperature was kept at 0 °C. The layers were separated and the organic solution was washed successively with 50 ml of an aqueous sodium hydroxide (25 wt%) solution and 300 ml of an aqueous hydrogen chloride (18 wt%) solution. The acidic extract was washed again with 100 ml diethylether and the unified organic fractions were dried over magnesium sulfate. Filtration and evaporation yielded 3.02 g (yield: 47 %) of a clear, yellowish oil.
IR(film, cm⁻¹) 2926/2855 (CH₃/CH₂), 1715 (CO), 1639 (C=C), 1461/1375 (CH₃/CH₂), 1265/1169/1101/1023/951
¹H-NMR (250 MHz, CDCl₃, ppm) 1.15-1.36 (m, 15H, CH₂,CH₃), 1.45-1.77 (m, 4H, CH₂), 3.41 (t, 2H, CH₂(3)), 4.03-4.24 (m, 4H, C*H*₂OPO₃H₂, OC*H*₂CH₃), 4.11 (s, 2H, CH₂(1)), 5.79 (s, 1H, C*H*=C), 6.22 (s, 1H, C*H*=C).
¹³C-NMR (63 MHz, CDCl₃, ppm) 13.98 (*C*H₃), 25.08, 25.93, 28.92, 29.19, 29.25, 29.30 and 29.45 (*C*H₂ (4-11)), 60.42 (*C*H₂CH₃), 68.63 and 70.86 (*C*H₂ (1) and *C*H₂ (3)), 125.08 (*C*=C-CO), 137.38 (C=*C*-CO), 165.69 (C=C-*C*O).

### Example 3

### Ethyl 2-[4-hydroxy-2-oxabutyl]acrylate (5)

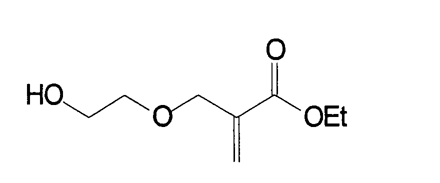

To a stirred solution of 3.56 g (57.35 mmol) 1,2-ethandiol and 5.86 g (57.90 mmol) triethylamine in 60 ml tetrahydrofurane 8.50 g (57.20 mmol) ethyl α-chloromethylacrylate were added at room temperature. The reaction mixture was refluxed for 28 h. The reaction mixture was cooled down to room temperature and the developing precipitate was filtered off. Evaporation of the filtrate afforded a clear, yellow oil. The crude product was solved in 150 ml dichloromethane and washed with 1x50 ml water. The organic layer was dried over magnesium sulfate the, filtered and evaporated. The crude product was purified by column chromatography on silica gel with dichloromethane as elution agent. This afforded 3.06 g (yield: 30,6 %) of a clear, yellowish oil.
IR(film, cm⁻¹) 3436 (OH), 2979, 2931, 2871 (CH₃/CH₂), 1710 (CO), 1638 (C=C), 1453/1373/1304 (CH₃/CH₂), 1270/1173/1109/1052/953.
¹H-NMR (250 MHz, CDCl₃, ppm) 1.27 (t, 3H, CH₃), 2.52 (broad s, 1H, OH), 3.54-3.63 (m, 2H, OCH₂CH₂O), 3.67-3.78 (m, 2H, OCH₂CH₂O), 4.15-4.24 (m, 4H, OCH₂ and C=C- CH₂) , 5.84 (s, 1H, C*H*=C), 6.28 (s, 1H, C*H*=C).
¹³C-NMR (63 MHz, CDCl₃, ppm) 14.06 (*C*H₃), 60.74 and 61.56 (*C*H₂ (4) and *C*H₂CH₃), 69.31 and 71.80 (*C*H₂ (1) and *C*H₂ (3)), 126.21 (*C*=C-CO), 137.03 (C=*C*-CO), 165.82 (C=C-*C*O).

### Ethyl 2-[5-dihydrogen phosphoryl-5,2-dioxabutyl]acrylate (6)

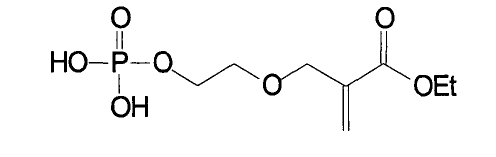

To a stirred solution of 2.94 g (19.21 mmol) phosphorusoxytrichloride in 50 ml diethylether a solution of 2.78 g (16.01 mmol) 5 and 1.95 g (19.31 mmol) triethylamine in 50 ml diethylether was added dropwise, while the temperature was kept at 0 °C. After the addition was finished the reaction mixture was stirred for 2 h at 0 °C and for 14 h at room temperature before the reaction was finished by filtration of the suspension and evaporation of the solvent. The oily residue was solved in 100 ml diethylether and added dropwise to 100 ml water, while the temperature was kept at 0 °C.

### Example 4

### (2,2 (4),4)-Trimethylhexamethylene bis(acrylamide)

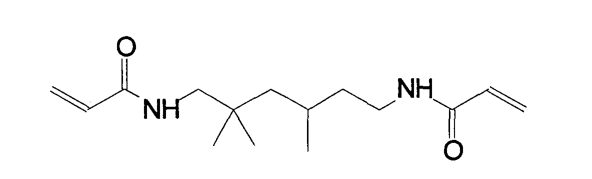

To a solution of 60 g (0.379 mol) (2,2 (4),4)-trimethylhexamethylene diamine in 100 ml methylene chloride a solution of 72.05 g (0.796 mol) acryloyl chloride in 130 ml methylene chloride and a solution of 31.84 g (0.796 mol) sodium hydroxide in 200 ml water were added simultaneously under stirring, so that the temperature remains at 0-5 °C. Thereafter the mixture was stirred at room temperature for additional two hours. The reaction was terminated by the addition of 100 ml water. The organic phase was separated and the aqueous solution was extracted twice with 30 ml methylene chloride. The combined organic liquids were washed with 150 ml of 1 n HCl, 150 ml of 1N NaHCO₃ and with 150 ml water. The solution of the product was stabilized with 0.025 mol% BHT and dried over sodium sulfate. Filtration and evaporation of the solvent yielded 87 g (yield: 86 %) of a colorless, highly viscose oil.
IR(film cm⁻¹) 3411, 3278 (NH), 2957 (CH2/CH3), 1656 (NHCO), 1546 (C=C), 1241 (CH2/CH3), 984/956 (C=C), 703 (C=C).
¹H-NMR (250 MHz, CDCl₃, ppm) 0.7 - 3.4 (several m, 18H, CH₃, CH₂), 5.51-5.60 (m, 1H, CH=C-CO), 6.16-6.26 (m, 2H, CH=CH-CO), 7.12, 6.84, 6.70, 6.59 (4 x t, 2H, NH).
¹³C-NMR (63 MHz, CDCl₃, ppm) 20.80, 22.21, 25.36, 26.01, 26.75, 27.90, 28.02, 29.04, 30.73, 32.80, 35.00, 35.68, 37.21, 38.79, 40.51, 45.16, 46.02, 47.02, 48.70, 125.66 (C=), 131.08 (C=), 165.82 (C=O), 166.09 (C=O).

## Claims

1. A polymerizable phosphoric acid ester derivative having the following formula (A): 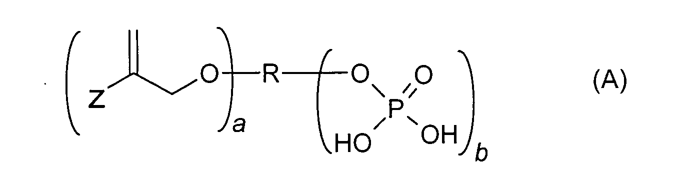 wherein
Z is COOR₁, COSR₁, CON(R₁)₂, CONR₁R₂, CONHR₁, or CN,
R₁ and R₂ independently are a hydrogen atom, a substituted or unsubstituted C₁ to C₁₈ alkyl group optionally substituted by a C₃ to C₈ cycloalkyl group, a substituted or unsubstituted C₃ to C₈ cycloalkyl group, a substituted or unsubstituted C₄ to C₁₈ aryl or heteroaryl group, a substituted or unsubstituted C₅ to C₁₈ alkylaryl or alkylheteroaryl group, or a substituted or unsubstituted C₇ to C₃₀ aralkyl group, whereby two R₁ residues may form together with the N-atom to which they are bonded a 5- to 7-membered heterocyclic ring which may contain beside said N-atom a further nitrogen atom or an oxygen atom, and whereby the substituted groups may be substituted by 1 to 5 C₁ to C₅ alkyl group(s);
*a* is an integer of from 1 to 10, preferably 1 to 5;
*b* is an integer of from 1 to 10, preferably 1 to 5; and
R represents an organic residue containing *a* + *b* carbon atoms selected from primary and secondary aliphatic carbon atoms, secondary alicyclic carbon atoms, and aromatic carbon atoms, each of said carbon atoms linking a phosphate or 2-(oxa-ethyl)acryl derivative group.

2. The polymerizable phosphoric acid ester derivative according to claim 1, wherein the organic residue R is
an (*a*+*b*)-valent saturated aliphatic C₂ to C₁₈ group having at least 2 of said primary aliphatic carbon atoms, and optionally 1 or more of said secondary aliphatic carbon atom(s), whereby said (*a*+*b*)-valent group may be substituted by C₁ to C₅ alkyl group(s); or
a C₂ to C₄₅ mono-, di-, or polyether which has from 1 to 14 oxygen atoms and is substituted by at least 2 C₁ to C₁₀ aliphatic group(s) having said primary and/or secondary aliphatic carbon atoms; whereby said ether may optionally be substituted by C₁ to C₅ alkyl group(s);
or
wherein the organic residue R represents:
a saturated C₃ to C₈ cyclic, C₇ to C₁₅ bi- or polycyclic hydrocarbon group having from 0 to 4, preferably, 0 to 3, more preferably 0 or 1, of said secondary alicyclic carbon atoms; and/or
a C₄ to C₁₈ aryl or heteroaryl group having from 0 to 5, preferably 0 to 3, more preferably 0 or 1, of said aromatic carbon atoms;
whereby said saturated hydrocarbon or aryl or heteroaryl group is substituted by
from 0 to 5 C₁ to C₅ alkyl group(s);
from 0 to 4, preferably 1 to 3, more preferably 1 or 2, saturated C₁ to C₁₀ aliphatic group(s) having said primary and/or secondary aliphatic carbon atoms, and/or
from 0 to 2 divalent residues according to one of the following formulas:
-[O-CH₂CH₂-]_{f}- wherein f is an integer of from 1 to 10, preferably 1 to 5;
-[-O-CH₂CH₂CH₂-]_{g}- wherein g is an integer offrom 1 to 10, preferably 1 to 5;
-[O-R₁₂]ₕ- wherein R₁₂ is -CH(CH₃)-CH₂- or -CH₂-CH(CH₃)- and h is an integer of from 1 to 10, preferably 1 to 5;
-[-O-R₁₄]ᵢ-[O-R₁₅]ⱼ- or -[O-R₁₅]ₖ-[O-R₁₄]ₗ- wherein R₁₄ is -CH₂CH₂-, R₁₅ is -CH(CH₃)-CH₂- or -CH₂-CH(CH₃)-, i, j, k, and l are integers whereby 2i + 3j ≤ 15 and 2k + 3l ≤ 15,
-[O-CH₂CH₂CH₂CH₂-]ᵣ- wherein r is an integer of 1 or 2;
wherein said divalent residues have one of said primary aliphatic carbon atoms; and
whereby 2 groups selected from said saturated hydrocarbon, aryl, and heteroaryl groups may optionally be linked by a single bond, an alkylene group, or -O-.

3. The polymerizable phosphoric acid ester derivative according to claim 1 or 2, wherein the organic residue R is
an (*a*+*b*)-valent saturated C₃ to C₈ cyclic or C₇ to C₁₅ bi- or tricyclic hydrocarbon group having at least 2 of said secondary alicyclic carbon atoms;
an (*a*+*b*)-valent saturated C₄ to C₁₈ aryl or heteroaryl group having from 2 to 6 of said aromatic carbon atoms;
an (*a*+*b*)-valent C₆ to C₁₈ alkylaryl or alkyl heteroaryl group having at least one of said aromatic carbon atoms, at least one of said secondary aliphatic carbon atoms, and optionally one of said primary aliphatic carbon atoms at the terminal end of the alkyl moiety of said alkylaryl or alkylheteroaryl group; or
an (*a*+*b*)-valent C₈ to C₃₀ aralkyl group having at least one of said primary aliphatic carbon atoms and at least one of said secondary aliphatic carbon atoms.

4. The polymerizable phosphoric acid ester derivative according to claim 1 or 2, **characterized by** one of the following formulas: 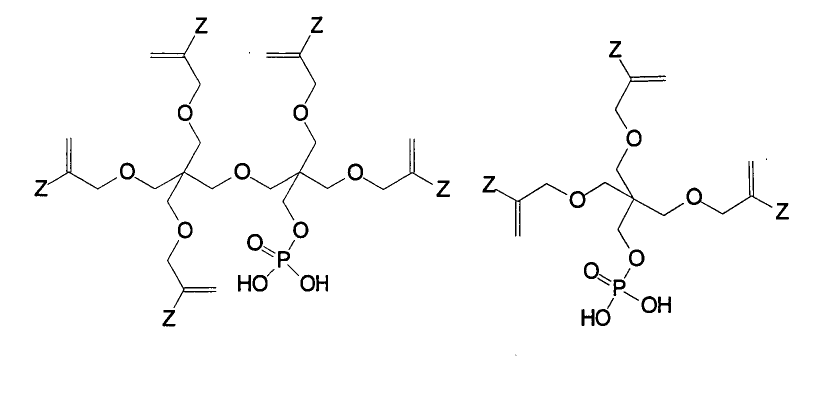 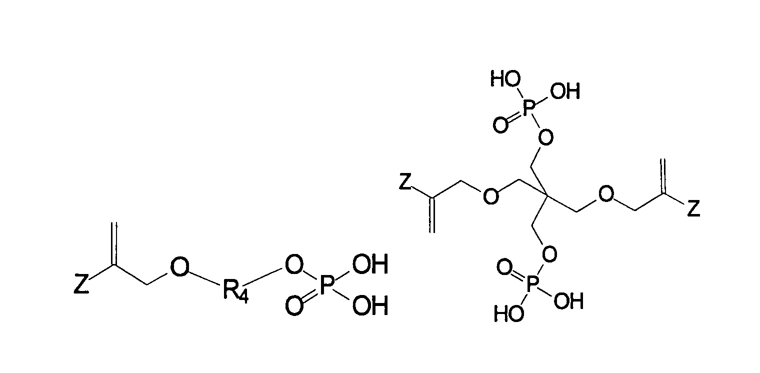 wherein
Z is as defined in claim 1,
R₄ denotes a divalent C₂ to C₁₈ alkylene group, a divalent C₃ to C₈ cycloalkylene group, a divalent C₄ to C₁₈ aryl or heteroaryl group, a divalent C₅ to C₁₈ alkylaryl or alkylheteroaryl group, a divalent C₇ to C₃₀ aralkyl group, whereby each of said groups may be substituted by 1 to 5 C₁ to C₅ alkyl group(s).

5. The polymerizable phosphoric acid ester derivative according to claim 4, wherein R₄ is a divalent residue according to one of the following formulas:
-[CH₂CH₂-O-]ₘ-CH₂CH₂- wherein m is an integer of from 1 to 14,
-[CH₂CH₂ CH₂-O-]ₚCH₂CH₂ CH₂- wherein p is an integer of from 1 to 14,
-[R₁₂-O]_{q}-R₁₃- wherein R₁₂ and R₁₃ may be -CH(CH₃)-CH₂- or
-CH₂-CH(CH₃)- and q is from 1 to 14,
-[R₁₄-O]ᵣ-[R₁₅-O]ₛ-R₁₄- or -[R₁₄-O]ₜ-[R₁₅-O]ᵤ-R₁₅- wherein R₁₄ is
-CH₂CH₂-, R₁₅ is -CH(CH₃)-CH₂- or -CH₂-CH(CH₃)-, r, s, t, and u are integers whereby 2r + 3s ≤ 43 and 2t + 3u ≤ 42,
-[CH₂CH₂CH₂CH₂-O-]ᵣ-CH₂CH₂CH₂CH₂- wherein r is 1 or 2, 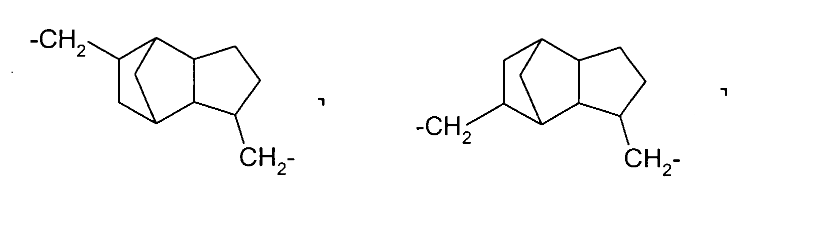 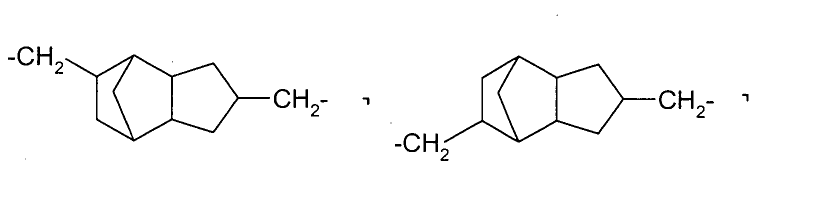 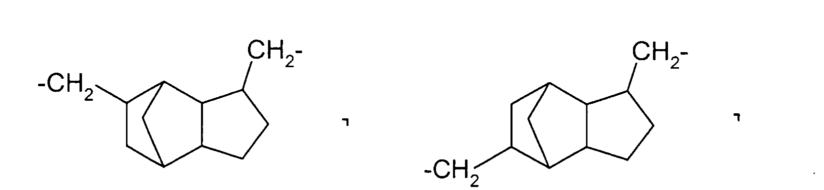 or 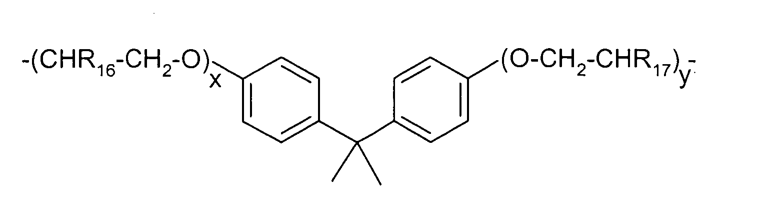 wherein R₁₆ and R₁₇ are H or -CH₃ and x and y may independently be integers of from 0 to 10, preferably 0 to 5.

6. The polymerizable phosphoric acid ester derivative according to any one of claims 1 to 5, wherein said (*a*+*b*) carbon atoms are primary aliphatic carbon atoms.

7. The polymerizable phosphoric acid ester derivative according to any one of claims 1 to 6, which is hydrolysis stable under acidic conditions, preferably at a pH of at most 4, more preferably at a pH of at most 2.

8. A method for producing the polymerizable phosphoric acid ester derivative according to any one of claims 1 to 7, which comprises the following steps:
(i) reacting a di- or polyol of the formula (HO)ₐ-R-(OH)_{b} (B) with a compound of the formula (C): 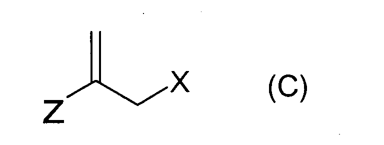 wherein Z, R, *a*, and *b* are as defined in any one of claims 1 to 5, particularly wherein R is R₄ as defined in claim 4 or 5, and
X is a leaving group for producing a compound (D) having *b* hydroxyl group(s) per molecule, and
(ii) reacting compound (D) with a phosphoric acid derivative (E) reactive with a hydroxyl group.

9. The method according to claim 8, wherein the equivalent ratio between compound (C) and the di- or polyol (B) is about *a* : 1.

10. The method according to claim 8 or 9, wherein the equivalent ratio between compounds (E) and (D) is about *b* : 1.

11. The method according to any one of claims 8 to 10, wherein the phosphoric acid derivative (E) is phosphorus trichloride oxide, and the method comprises hydrolyzing the reaction product of compounds (D) and (E).

12. The method according to any one of claims 8 to 11, wherein *b* hydroxyl groups of the polyol (B) are protected, then reacted with compound (C) followed by deprotecting before conducting step (ii).

13. Use of the polymerizable phosphoric acid ester derivative according to any one of claims 1 to 7 in a dental composition.

14. A dental composition comprising the polymerizable phosphoric acid ester derivative of any one of claims 1 to 7.

15. The dental composition of claim 14, which is acidic pH, preferably it has a pH of at most 4, more preferably a pH of at most 2, most preferably a pH of about 1.0.

16. The dental composition of claim 14 or 15, which further comprises a curing system.

17. The dental composition according to claim 16, wherein the curing system comprises a polymerization initiator, an inhibitor, and a stabilizer.

18. The dental composition according to any one of claims 14 to 17, which further comprises an organic water soluble solvent and/or water.

19. The dental composition according to any one of claims 14 to 18, which further comprises an organic and/or inorganic acid.

20. The dental composition according to any one of claims 14 to 19, which further comprises a polymerizable N-substituted alkylacrylic or acrylic acid amide monomer.

21. The dental composition according to claim 20, wherein the polymerizable N-substituted alkylacrylic or acrylic acid amide monomer is **characterized by** the following formulas: 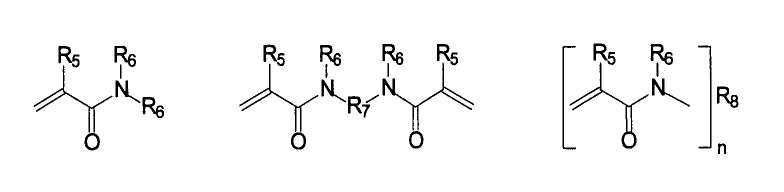 wherein
R₅ and R₆ independently represent an hydrogen atom, a substituted or unsubstituted C₁ to C₁₈ alkyl group, a substituted or unsubstituted C₃ to C₁₈ cycloalkyl group, a substituted or unsubstituted C₄ to C₁₈ aryl or heteroaryl group, a substituted or unsubstituted C₅ to C₁₈ alkylaryl or alkylheteroaryl group, a substituted or unsubstituted C₇ to C₃₀ aralkyl group, whereby two R₆ residues may form together with the N-atom to which they are bonded a 5-to 7-membered heterocyclic ring which may contain beside said N-atom a further nitrogen atom or an oxygen atom; and whereby the substituted groups may be substituted by 1 to 5 C₁ to C₅ alkyl group(s); R₇ denotes a divalent substituted or unsubstituted organic residue having from 1 to 45 carbon atoms, whereby said organic residue may contain from 1 to 14 oxygen and/or nitrogen atoms and is selected from
a C₁ to C₁₈ alkylene group wherein from 1 to 6 -CH₂-groups may be replaced by a -N-(C=O)-CR₉=CH₂ group wherein R₉ is a hydrogen atom or a C₁ to C₁₈ alkyl group,
a divalent substituted or unsubstituted C₃ to C₁₈ cycloalkyl or cycloalkylene group,
a divalent substituted or unsubstituted C₄ to C₁₈ aryl or heteroaryl group, a divalent substituted or unsubstituted C₅ to C₁₈ alkylaryl or alkylheteroaryl group,
a divalent substituted or unsubstituted C₇ to C₃₀ aralkyl group, and
a divalent substituted or unsubstituted C₂ to C₄₅ mono-, di- or polyether group having from 1 to 14 oxygen atoms,
R₈ denotes a saturated di- or multivalent substituted or unsubstituted C₂ to C₁₈ hydrocarbon group, a saturated di- or multivalent substituted or unsubstituted cyclic C₃ to C₁₈ hydrocarbon group, a di- or multivalent substituted or unsubstituted C₄ to C₁₈ aryl or heteroaryl group, a di- or multivalent substituted or unsubstituted C₅ to C₁₈ alkylaryl or alkylheteroaryl group, a di- or multivalent substituted or unsubstituted C₇ to C₃₀ aralkyl group, or a di- or multivalent substituted or unsubstituted C₂ to C₄₅ mono-, di-, or polyether residue having from 1 to 14 oxygen atoms,
n is an integer, preferably from 2 to 10, more preferably from 3 to 4.

22. The dental composition according to claim 21, wherein said polymerizable monomer is a mono-, bis- or poly(meth) acrylamide **characterized by** one of the following formulas:

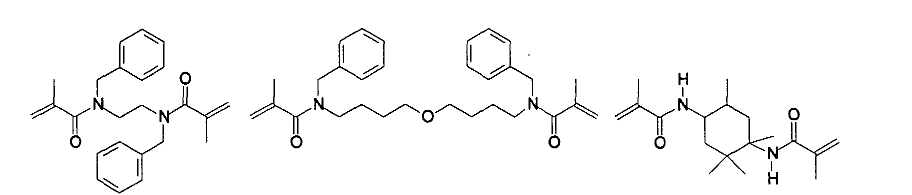

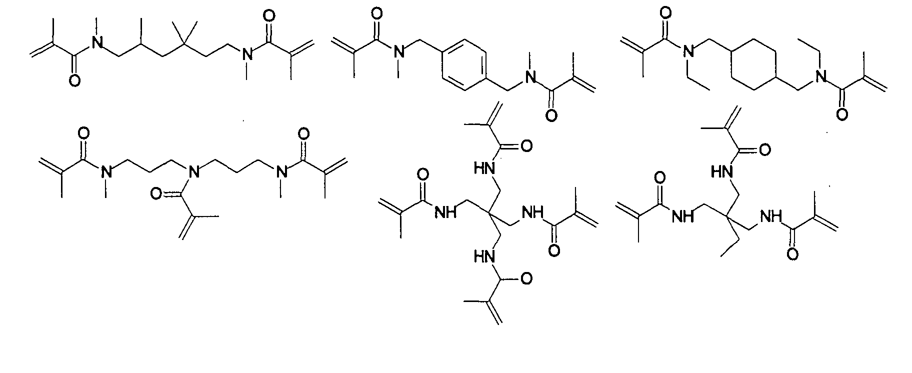

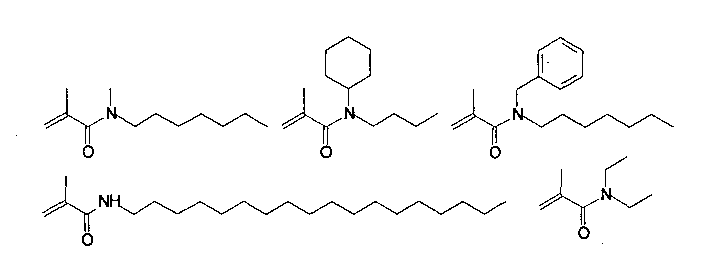

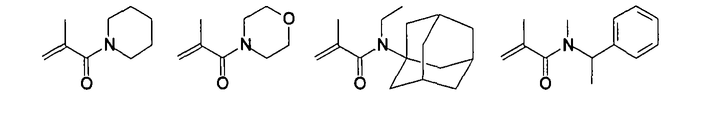

23. The dental composition according to any one of claims 14 to 22, which is a hydrolysis stable one-part self-etching, self-priming dental adhesive composition.

24. The dental composition according to any one of claims 14 to 23, which is hydrolysis stable for at least one week at a storage temperature of 50 °C, whereby after such storage the bond strength of an adhesive prepared from such a dental composition to enamel and/or dentin is at least 10 MPa, preferably 15 MPa.

25. The dental composition according to any one of claims 14 to 24, which contains from 5 to 90 wt-% of the polymerizable phosphoric acid ester derivative according to claim 1.
